# EUROPEAN PATENT APPLICATION

(11) **EP 1 779 832 A1**
(43) Date of publication of application: **02.05.2007**
(21) Application number: 05767210.7
(22) Date of filing: 26.07.2005
(51) Int. Cl.: A61J 1/05, B65D 1/32, B65D 83/00

(54) **SQUEEZE BOTTLE AND EYE DROP CONTAINER USING THE SAME**

(30) Priority: 30.07.2004 JP 2004224578
(71) Applicant: OTSUKA PHARMACEUTICAL CO., LTD., Chiyoda-ku Tokyo 101-8535 (JP); Otsuka Techno Corporation, Naruto-shi, Tokushima 772-0017 (JP)
(72) Inventor: KAWASHIRO, Yasushi, Anan-shi, Tokushima 7740011 (JP); SUGAHARA, Yuji, 1-36, Nakanokoshi, Itano-gun, Tokushima 7710212 (JP); YAMAZAKI, Hiroyuki, Tokushima-shi, Tokushima 7710130 (JP)
(74) Representative: HOFFMANN EITLE
(86) International application number: PCT/JP2005/013638
(87) International publication number: WO 2006/011473

(57) **Abstract**

To provide a squeeze bottle with reduced pressing force required to delivery a liquid, and particularly a squeeze bottle wherein the pressing force is reduced to such an extent that allows easy control of the discharge of liquid drops even when a valve operated by the liquid pressure is provided.

In a squeeze bottle comprising the bottle body 10 for containing a liquid, and a neck 11 that leads to the opening end 10a of the bottle body 10, the bottle body 10 has a bellows 12 which is contracted and expanded in the direction y generally perpendicular to the direction x of delivering liquid. The bellows 12 has a plurality of pleated portions 13 disposed in the direction of delivery x. The pleated portions 13 are disposed at intervals in the direction of contraction y. Further, the width of the convex edge of the pleated portion 13 in the direction of width Z, that is, the distance between the both edges 14, is made to decrease from the middle to the both ends in the direction of contraction y. With respect to the concave edge of the pleated portion 13, the distance between the both edges 18 in the direction of width z is also made to decrease from the middle to the both ends in the direction of contraction y.

## Description

### Technical Field

The present invention relates to a squeeze bottle with reduced pressing force required to delivery a liquid, and to an eye dropper that uses the same.

### Background Art

An ophthalmic solution contained in an eye dropper includes an antiseptic agent added therein so as to prevent the liquid from becoming corrupt, degenerating or otherwise degrading. However, there are such problems that some types of ophthalmic solution prohibit the addition of an antiseptic agent, and that the antiseptic agent may become an allergen. Accordingly, it has been called for to either discontinue the use of the antiseptic agent or significantly reduce the quantity thereof.

The present applicant previously proposed a delivery container provided with, at a mouth of the container body, a delivery port having a valve that operates in response to liquid pressure (Patent Document 1). With this container, when the container body is pressed so as to deform the valve that blocks the delivery port by means of liquid pressure, a liquid drop is delivered. When the pressure applied to the container body is removed, the valve restores itself to the original shape and the delivery port is blocked up, so that backflow of the discharged liquid and infiltration of microorganisms and the like from the outside of the container can be prevented. Accordingly, use of this container as an eye dropper enables it to either discontinue the use of the antiseptic agent or significantly reduce the quantity thereof.

Meanwhile there is a demand for such a container having a delivery port with a valve, to reduce the pressing force required to discharge a liquid drop. Moreover, there is such a demand that it should be easy to control the force of pressing the container body so as to freely control the discharge of liquid drops, specifically, it should be easy to control the liquid drop to be delivered one by one, while preventing the problem of continuous flow of the liquid under an excessive force.

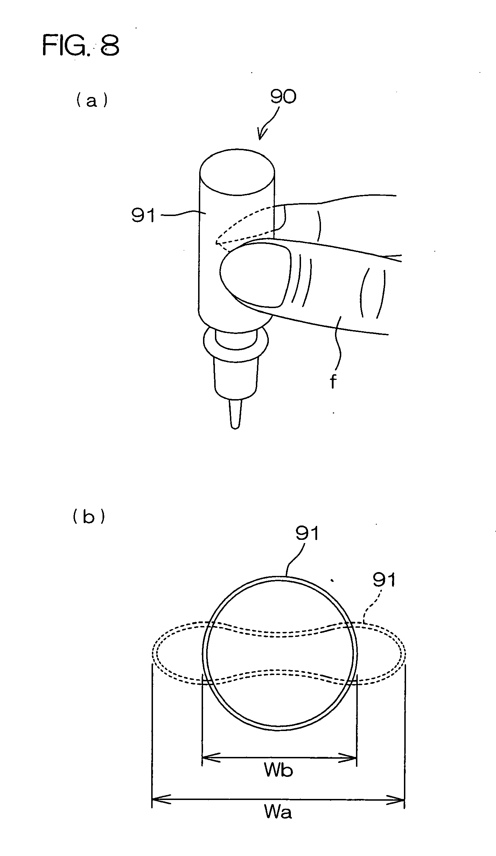

However, for example, in the case of a cylindrical squeeze bottle 90, since a width Wa of plane of projection of a bottle body 91 when the bottle body 91 is pressed and deformed is larger than a width Wb of the plane of projection before the bottle body 91 is pressed (Fig. 8), the force which fingers f receive (reactive force) significantly increases as the bottle body 91 is deformed. As a result, in case the squeeze bottle 90 such as shown in Fig. 8 is provided with the delivery port having the valve described above, the bottle is pressed until the activating pressure of the valve is attained, thus inevitably resulting in the state of Wa > Wb. Therefore, in this case, it is difficult to reduce the force required to press the bottle body.

On the other hand, patent document 2 describes an application tool having at least one bellows that has folding lines running vertically, provided on a side surface of the container body.
[Patent Document 1] WO2004/011345 A1
[Patent Document 2] Japanese Unexamined Patent Publication No. 2000-107673

### Disclosure of the Invention

### Problems to be Solved by the Invention

In the container body of the application tool described in Patent Document 2, the pressing surface of the pleated portions disposed on both ends of the bellows in the contracting direction thereof are formed with a width comparable to that of the container body, so that projection of the pleated portion that constitutes the pressing surface onto a plane perpendicular to the direction of contraction substantially coincides with the projection of the container body onto a plane perpendicular to the direction of contraction (refer to Figs. 1 to 8 of said Patent Document). Therefore, the problem that the force which fingers receive increases as the container body is deformed when the container body is pressed with fingers can be prevented.

However, in the application tool described in Patent Document 2, a width of the pleated portion, which forms the bellows, in direction perpendicular to the contracting direction of the bellows, is substantially constant between the middle and ends along the contracting direction. As a result, when the container body is pressed for compression, pleated portions are put one on another, thus generating a large reactive force.

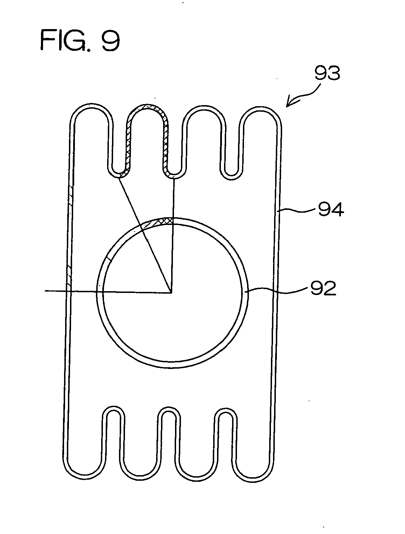

Further, in case the container body is formed by blow molding, a parison 92 is expanded with greatly different expansion ratios depending on the portion thereof that would make different wall portions 94 of the container body 93 (refer to Fig. 9 of the present invention). This results in such problems as wall thickness of the container body varies from one portion to another or it becomes difficult to form the container body by blow molding.

Accordingly, an object of the present invention is to provide a squeeze bottle with reduced force of pressing the container body required to deliver a liquid, and particularly a squeeze bottle that allows it to easily control the discharge of liquid drops, even when a valve operated by the liquid pressure is provided in the delivery port of the container that utilizes the squeeze bottle.

### Means for Solving the Problems

In order to achieve the object described above, the present invention provides the following devices:
(1) a squeeze bottle comprising a bottle body for containing a liquid, and a neck that leads to an opening end of the bottle body, wherein the bottle body has a bellows that contracts and expands in a direction generally perpendicular to the direction of delivering the liquid that is discharged through the neck, the bellows has a plurality of pleated portions disposed in the direction of delivery, each pleated portion is disposed at intervals from each other in the direction of contraction, the width of the pleated portion at the convex edge in a direction perpendicular to the direction of contraction is made to decrease from the middle to the both ends in the direction of contraction, and
   the width of the pleated portion at the concave edge in the direction perpendicular to the direction of contraction is made to decrease from the middle to the both ends in the direction of contraction;
(2) the squeeze bottle according to above (1), wherein the both edges at the convex edges of the pleated portion in the direction perpendicular to the contracting direction are disposed generally along a circle when viewed in the direction of delivery, and the both edges at the concave edges of the pleated portion in the direction perpendicular to the contracting direction are disposed along a generally concentric circle of a radius smaller than that of the former circle when viewed in the direction of delivery;
(3) the squeeze bottle according to above (1), wherein the bottle body has a cylindrical portion that adjoins the bellows in the direction of delivery, the both edges at the convex edges of the pleated portion in the direction perpendicular to the contracting direction are disposed along the circumferential surface of the cylindrical portion when viewed in the direction of delivery, and the both edges at the concave edges of the pleated portion in the direction perpendicular to the contracting direction are disposed along a generally concentric circle of a radius smaller than the circumference of the cylindrical portion when viewed in the direction of delivery;
(4) the squeeze bottle according to above (1) that is formed by blow molding;
(5) the squeeze bottle according to above (1), which is provided with a delivery member that is connected detachably to the neck and has a valve operated by liquid pressure; and
(6) an eye dropper that utilizes the squeeze bottle according to above (1).

### Effect of the Invention

With the squeeze bottle of the present invention, since the bottle body is provided with the bellows, fingers that press the bottle body do not receive reactive force from the entire bottle body.

The bellows has a plurality of pleated portions disposed in the direction of delivery of the squeeze bottle, while the widths of the convex edges of the pleated portions perpendicular to the contracting direction decreases from the middle toward the both ends in the contracting direction, and widths of the concave edges of the pleated portions perpendicular to the contracting direction also decreases from the middle toward the both ends in the contracting direction, so that the pleated portions are not put one on another so much when the container body is pressed to deform, thus reducing the reactive force received by the fingers. Moreover, with the constitution described above, since the projection of the pleated portion, located at both ends among the pleated portions in the direction of contraction, onto a plane perpendicular to the direction of contraction falls within the projection of a pleated portion, other than the pleated portions located at the both ends, onto a plane perpendicular to the direction of contraction, the pressing force required to deliver the liquid from the squeeze bottle can be greatly reduced.

### Brief Description of the Drawings

[Fig. 1] One embodiment of the squeeze bottle according to the present invention, (a) being a partially cut-away front view, (b) being a side view and (c) being a sectional view taken along lines A-A in (a).
[Fig. 2] A front view of the squeeze bottle shown in Fig. 1 having a delivery member provided with a valve.
[Fig. 3] A front view showing an example of modifying the design of the squeeze bottle shown in Fig. 1.
[Fig. 4] Another embodiment of the squeeze bottle according to the present invention, (a) being a partially cut-away front view, (b) being a side view and (c) being a bottom view.
[Fig. 5] A front view of the squeeze bottle shown in Fig. 4 having a delivery member provided with a valve.
[Fig. 6] A further another embodiment of the squeeze bottle according to the present invention, (a) being a partially cut-away front view, (b) being a side view and (c) being a sectional view taken along lines B-B in (a).
[Fig. 7] A front view of the squeeze bottle shown in Fig. 6 having a delivery member provided with a valve.
[Fig. 8] (a) is a perspective view showing an example of conventional squeeze bottle, and (b) is a schematic diagram showing a body of the squeeze bottle being pressed.
[Fig. 9] A schematic cross sectional view showing the relationship between a conventional container having a bellows and a parison used in forming the same.

### Description of Reference Numerals

- 10, 20, 30:: Bottle body
- 10a, 20a, 30a:: Opening end
- 11, 21, 31:: Neck
- 12, 22, 32:: Bellows
- 13, 23, 33:: Pleated portion
- 14, 24, 34:: Both edges
- 16, 36:: Cylindrical portion
- 17, 37:: Circumferential surface
- 80, 80':: Delivery member
- 81:: Valve

### Best Mode for Carrying Out the Invention

Preferred embodiments of the present invention will now be described in detail with reference to the accompanying drawings.

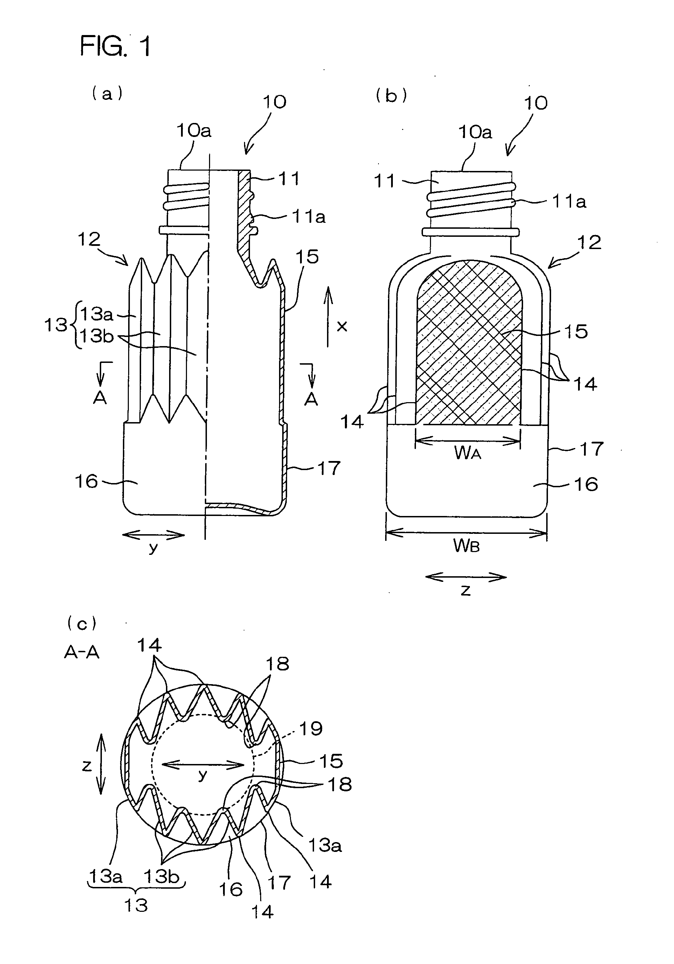

Fig. 1(a) to Fig. 1(c) show a first embodiment of the squeeze bottle according to the present invention. The squeeze bottle shown in Fig. 1 comprises a bottle body 10 for containing a liquid, and a neck 11 that leads to an opening end 10a of the bottle body.

The bottle body 10 has a bellows 12 which can be freely contracted and expanded in a direction (direction of contraction y) generally perpendicular to the direction x of delivering liquid from the neck 11 of the bottle body 10. The bellows 12 has a plurality of pleated portions 13 disposed in the direction of delivery x. The pleated portions 13 are disposed at intervals in the direction of contraction y of the bellows 12. The bottle body 10 further comprises a cylindrical portion 16 that is disposed adjacent to the bellows 12 in the direction of delivery x.

In the squeeze bottle shown in Fig. 1, the projection of the pleated portions 13a, located at both ends in the direction of contraction y among the pleated portions 13 that form the bellows 12, onto a plane perpendicular to the direction of contraction y falls within the projection of any pleated portion 13b, other than the pleated portions 13a onto a plane perpendicular to the direction of contraction y.

Moreover, both edges 14 at the convex edge of the pleated portion 13 in a direction (direction of width) z perpendicular to the direction of contraction y are disposed along the circumferential surface 17 of the cylindrical portion 16, and width of the convex edge in the direction of width z, namely the distance between the both edges 14 is made to decrease from the middle to the ends in the direction of contraction y.

Both edges 18 at the concave edge of the pleated portion 13 in the direction of width z are also disposed substantially along a circle 19 shown in Fig. 1, and the distance between the both edges 18 is made to decrease from the middle to the ends in the direction of contraction y.

Therefore, in the squeeze bottle shown in Fig. 1, projection A (indicated by hatching in Fig. 1(b)) of a pair of surfaces 15 (hereinafter referred to as pressing surface) of the bellows 12 located at both ends thereof in the direction of contraction y, which serves as the pressing surface of the squeeze bottle, onto a plane perpendicular to the direction of contraction y is made smaller than the projection B of any pleated portion 13b (not shown), other than the pleated portion 13a located at both ends in the direction of contraction y, onto a plane perpendicular to the direction of contraction y.

While width of the projection A is denoted by W_{A} and width of the projection B which has the largest area is denoted by W_{B} in Fig. 1(b), width of the projection A of the pressing surface 15 is smaller than the width of the projection B as can be seen from the drawing and, furthermore, the projection A is smaller than the projection of the cylindrical portion 16 onto a plane perpendicular to the direction of contraction y.

As a result, area of the pressing surface 15 can be prevented from increasing when the pressing surface 15 is pressed so as to deform the bottle body 10, and the pressing force required to deform the bottle body 10 can also be restricted from increasing.

Further, according to the squeeze bottle shown in Fig. 1, the both edges 18 at the concave edges of the pleated portions 13 are not to be put one on another so much when the pressing surface 15 is pressed to deform, thus reducing the reactive force received by the fingers.

Moreover, according to the squeeze bottle shown in Fig. 1, since widths of the convex edges and concave edges of the pleated portions 13 in the direction (direction of width) z perpendicular to the direction of contraction y is made to decrease from the middle to the ends in the direction of contraction y, as described above, it is prevented that difference in expansion ratios of a parison and difference in wall thickness after molding depending on the wall portion of the container body are made, when the container body is formed by blow molding. Therefore, the squeeze bottle shown in Fig. 1 is advantageously formed by blow molding.

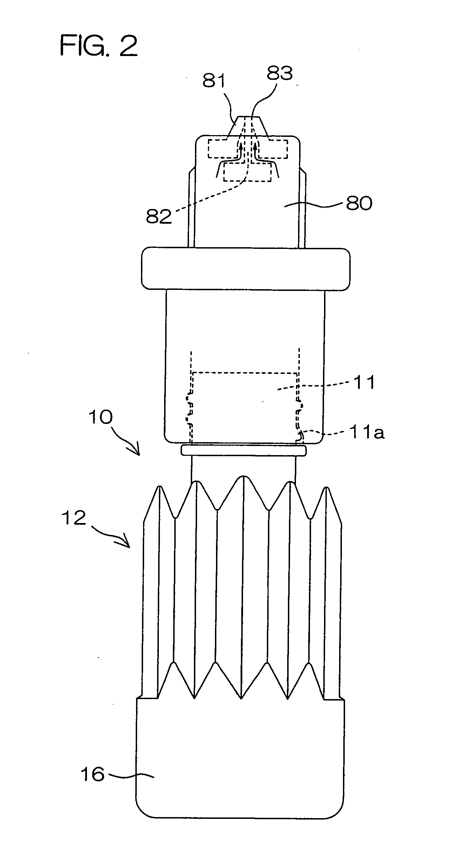

Fig. 2 shows the neck 11 of the squeeze bottle shown in Fig. 1 provided with a delivery member 80 having a valve 81 operated by the liquid pressure connected detachably thereto.

While there are no limitations on the shape, structure, method of operation and the like of the valve 81 of the delivery member 80, various known valves including, for example, the valve described in Patent Document 1 may be used. In the case of the delivery member 80 shown in Fig. 1, the valve 81 normally makes contact with a valve support member 82 to block a delivery orifice 83. On the other hand, when the bottle body 10 is pressed to apply a liquid pressure to the valve 81, the valve 81 opens toward the outside so that a clearance is formed in relation to the valve support member 82. As a result, the liquid drop can be delivered through the delivery orifice 83. When the liquid pressure applied to the valve 81 is removed, the valve 81 restores the original shape to close the delivery orifice 83, thereby preventing the back flow of the liquid.

In the squeeze bottle shown in Fig. 2, the delivery member 80 is fastened by being screwed onto a threaded portion 11a formed on the neck 11 of the bottle body 10. The means of fastening the delivery member is not limited to this, and it may be fastened by simply engaging the delivery member and the neck of the bottle to each other.

According to the squeeze bottle shown in Fig. 2, while the delivery member 80 having the valve 81 operated by the liquid pressure is provided, liquid drops can be delivered with a small force that can be freely controlled. Further, when the squeeze bottle shown in Fig. 2 is used as an eye dropper, the ophthalmic solution can be easily delivered with a small pressure by means of two fingers, for example, a thumb and an index finger, while the quantity of the liquid delivered can be easily controlled.

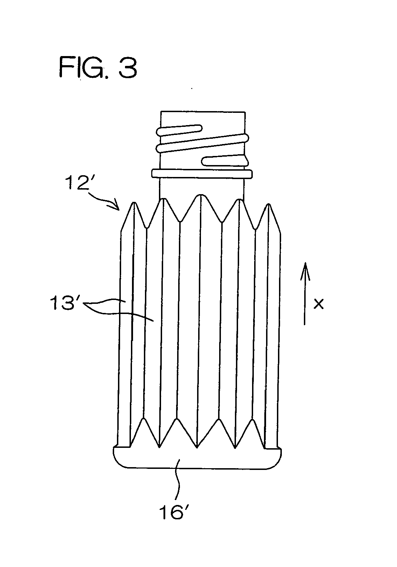

Fig. 3 is a front view showing an example of modifying the design of the squeeze bottle shown in Fig. 1. In the first embodiment described above, length of a bellows 12' in the direction of delivery x is not restricted, but a pleated portion 13' may extend to near the bottom of the bottle body (cylindrical portion 16') shown in Fig. 3.

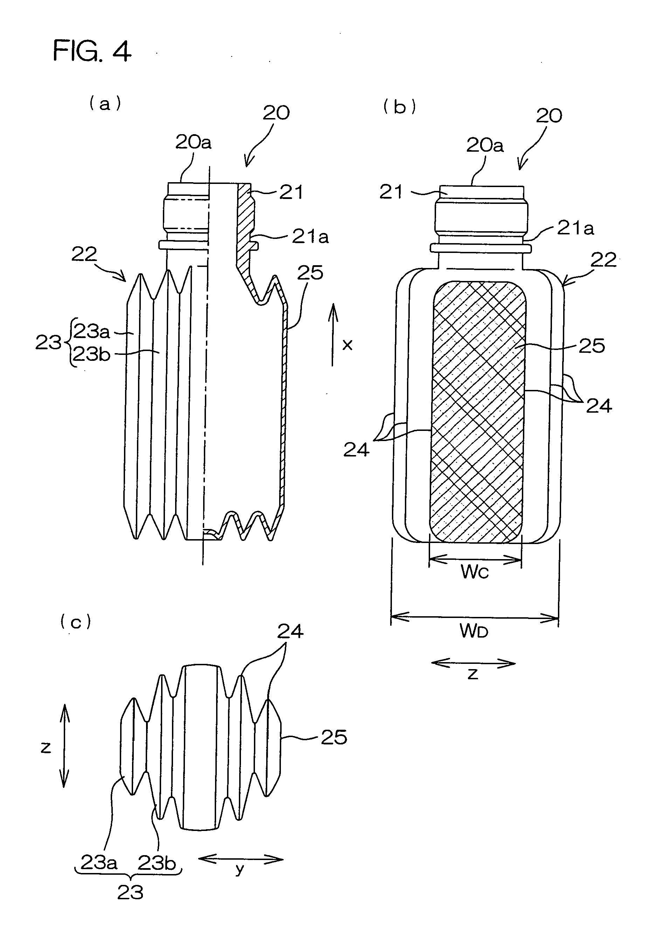

Fig. 4(a) to Fig. 4(c) show a second embodiment of the squeeze bottle according to the present invention. As shown in Fig. 4, the squeeze bottle is provided with a bottle body 20 for containing a liquid, and a neck 21 that leads to an opening end 20a of the bottle body 20, similarly to the first embodiment.

The bottle body 20 has a bellows 22 which can be freely contracted and expanded in the direction (direction of contraction y) generally perpendicular to the direction x of delivering liquid from the neck 21 of the bottle body 20. The bellows 22 has a plurality of pleated portions 23 disposed in the direction of delivery x. The pleated portions 23 are disposed at intervals in the direction of contraction y of the bellows 22.

In the squeeze bottle shown in Fig. 4, projection of the pleated portion 23a, located at both ends in the direction of contraction y, among the pleated portions 23 that form the bellows 22, onto a plane perpendicular to the direction of contraction y falls within the projection of any pleated portion 23b, other than the pleated portion 23a, onto a plane perpendicular to the direction of contraction y.

Moreover, distance between the both edges 24 at the convex edge of the pleated portions 23 in the direction (direction of width) z perpendicular to the contracting direction y is made to decrease from the middle to the ends in the direction of contraction y, and distance between the both edges at the concave edge of the pleated portions 23 is also made to decrease from the middle to the ends in the direction of contraction y.

Therefore, in the squeeze bottle shown in Fig. 4, projection C (indicated by hatching in Fig. 4(b)) of a pair of surfaces 25 (hereinafter referred to as pressing surface) of the bellows 22 located at both ends thereof in the direction of contraction y, which serves as the pressing surface of the squeeze bottle, onto a plane perpendicular to the direction of contraction y is smaller than the projection D (not shown) of any pleated portion 23b, other than pleated portion 23a located at both ends in the direction of contraction y, onto a plane perpendicular to the direction of contraction y.

While width of the projection C is denoted by W_{C} and width of the projection D which has the largest area is denoted by W_{D} in Fig. 4(b), width of the projection C of the pressing surface 25 is smaller than the width of the projection D, as is clear from this figure.

As a result, area of the pressing surface 25 can be restricted from increasing when the pressing surface 25 is pressed to deform the bottle body 20, and the pressing force required to deform the bottle body 20 can also be restricted from increasing.

Further, according to the squeeze bottle shown in Fig. 4, both edges at the concave edges of the pleated portions 23 are not likely to be put one on another so much when the pressing surface 25 is pressed to deform, thus reducing the reactive force received by the fingers.

Moreover, according to the squeeze bottle shown in Fig. 4, since widths of the convex edges and concave edges of the pleated portions 23 in the direction (direction of width) z perpendicular to the direction of contraction y is made to decrease from the middle to the ends in the direction of contraction y, as described above, it is prevented that difference in expansion ratios of a parison and difference in wall thickness after molding depending on the wall portion of the container body are made, when the container body is formed by blow molding. Therefore, the squeeze bottle shown in Fig. 4 is advantageously formed by blow molding.

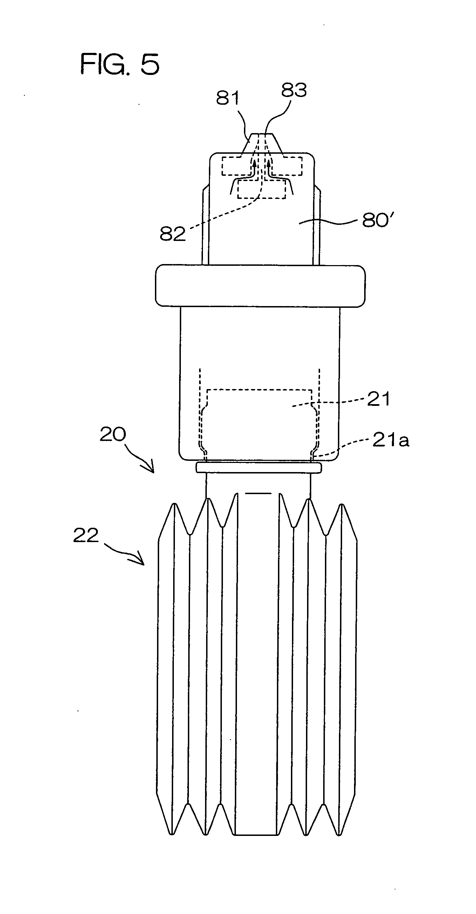

Fig. 5 shows the neck 21 of the squeeze bottle shown in Fig. 4 provided with a delivery member 80' having the valve 81 operated by the liquid pressure connected detachably thereto.

The shape, structure, method of operation and the like of the valve 81 of the delivery port 80' are similar to those of the first embodiment.

In the squeeze bottle shown in Fig. 5, the delivery member 80' is fastened by fitting an engagement portion 21a provided on the neck 21 of the bottle body 20 with a protruding portion formed on the inner circumference of the delivery portion 80'.

According to the squeeze bottle shown in Fig. 5, while the delivery member 80 having the valve 81 operated by the liquid pressure is provided, liquid drops can be delivered with a small force that can be freely controlled. Further, when the squeeze bottle shown in Fig. 5 is used as eye dropper, the ophthalmic solution can be easily delivered with a small pressure by means of two fingers, for example, a thumb and an index finger, while the quantity of the liquid delivered can be easily controlled.

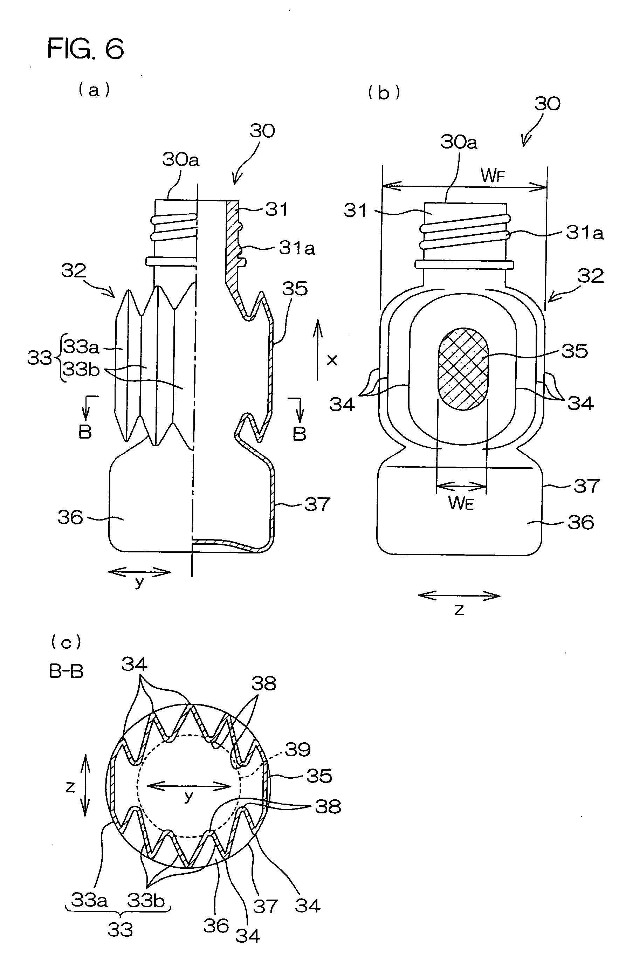

Fig. 6(a) to Fig. 6(c) show a third embodiment of the squeeze bottle according to the present invention. As shown in Fig. 6, the squeeze bottle is provided with the bottle body 30 for containing a liquid, and a neck 31 that leads to an opening end 30a of the bottle body 30, similarly to the first and second embodiment.

The bottle body 30 has a bellows 32 which can be freely contracted and expanded in the direction (direction of contraction y) generally perpendicular to the direction x of delivering liquid from the neck 31 of the bottle body 30. The bellows 32 has a plurality of pleated portions 33 disposed in the direction of delivery x. The pleated portions 33 are disposed at intervals in the direction of contraction y of the bellows 32.

In the squeeze bottle shown in Fig. 6, projection of the pleated portion 33a, located at both ends in the direction of contraction y, among the pleated portions 33 that form the bellows 32, onto a plane perpendicular to the direction of contraction y falls within the projection of any pleated portion 33b, other than the pleated portion 33a, onto a plane perpendicular to the direction of contraction y.

Moreover, the both edges 34 of the pleated portions 33 at the convex edge in the direction (direction of width) z perpendicular to the direction of contraction y are disposed along circumferential surface 37 of the cylindrical portion 36, and width of the convex edge in the direction of width z, namely the distance between the both edges 34, is made to decrease from the middle to the both ends in the direction of contraction y.

The both edges 38 of the pleated portions 33 at the concave edge in the direction of width z are also disposed along a circle 39 shown in Fig. 6, and the distance between the both edges 38 is made to decrease from the middle to the ends in the direction of contraction y.

Therefore, in the squeeze bottle shown in Fig. 6, projection E (indicated by hatching in Fig. 6(b)) of a pair of surfaces 35 (hereinafter referred to as pressing surface) of the bellows 32 located at both ends thereof in the direction of contraction y, which serves as the pressing surface of the squeeze bottle, onto a plane perpendicular to the direction of contraction y is smaller than the projection F (not shown) of any pleated portion 33b, other than pleated portion 33a located at both ends in the direction of contraction y, onto a plane perpendicular to the direction of contraction y.

While width of the projection E is denoted by W_{E} and width of the projection F which has the largest area is denoted by W_{F} in Fig. 6(b), width W_{E} of the projection E of the pressing surface 35 is smaller than the width W_{F} of the projection F, as is clear from this figure.

As a result, area of the pressing surface 35 can be restricted from increasing when the pressing surface 35 is pressed to deform the bottle body 30, and the pressing force required to deform the bottle 30 can also be restricted from increasing.

Further, according to the squeeze bottle shown in Fig. 6, both edges 38 at the concave edges of the pleated portions 33 are not to be put one on another so much when the pressing surface 35 is pressed to deform, thus reducing the reactive force received by the fingers.

Moreover, according to the squeeze bottle shown in Fig. 6, since widths of the convex edges and the concave edges of the pleated portions 33 in the direction (direction of width) z perpendicular to the direction of contraction y is made to decrease from the middle to the both ends in the direction of contraction y, as described above, it is prevented that difference in expansion ratios of a parison and difference in wall thickness after molding depending on the wall portion of the container body are made, when the container body is formed by blow molding. Therefore, the squeeze bottle shown in Fig. 6 is advantageously formed by blow molding.

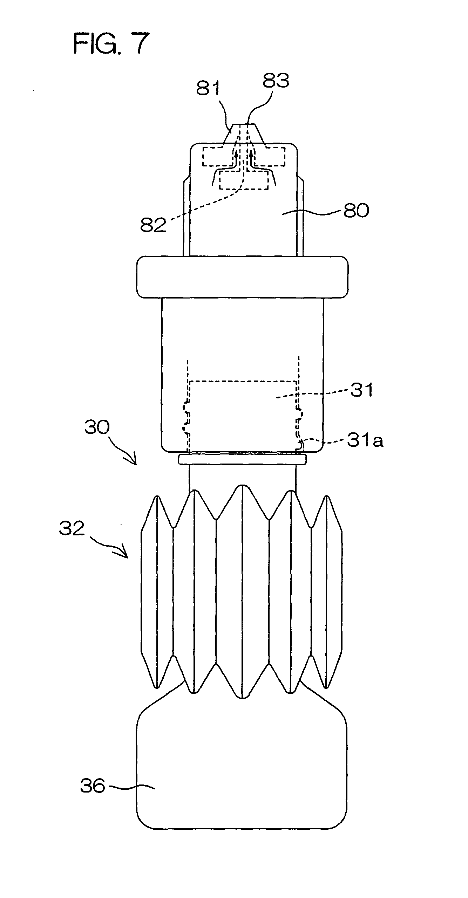

Fig. 7 shows the neck 31 of the squeeze bottle shown in Fig. 6 provided with the delivery member 80 having the valve 81 operated by the liquid pressure connected detachably thereto.

The shape, structure, method of operation and the like of the valve 81 of the delivery member 80 are similar to those of the first embodiment. Further, the delivery member 80 is fastened by being screwed onto a threaded portion 31a formed on the neck 31 of the bottle 30, similarly to the first embodiment.

According to the squeeze bottle shown in Fig. 7, while the delivery member 80 having the valve 81 operated by the liquid pressure is provided, liquid drops can be delivered with a small force that can be freely controlled. When the squeeze bottle shown in Fig. 7 is used as an eye dropper, the ophthalmic solution can be easily delivered with a small pressure by means of two fingers, for example, a thumb and an index finger, while the quantity of the liquid delivered can be easily controlled.

While there is no restriction on the material used to form the bottle body of the squeeze bottle according to the present invention, a thermoplastic resin is preferably used in order to form the entire bottle body including the bellows and the neck as a single piece. In case a liquid medicine such as ophthalmic medicine is contained in the bottle body, it is preferable to form the bottle body from a polyolefin such as polyethylene or polypropylene that is approved for use in medical applications.

There are no particular requirements on a liquid contained in the bottle, except that the liquid should have fluidity, specifically the liquid must be able to move from within the bottle toward the valve when the pressing surface of the squeeze bottle is pressed, so that liquid pressure can be applied to the valve in the delivery member whereby the liquid is delivered through the delivery orifice to the outside of the squeeze bottle. Further, there are no restriction on the composition and physical properties including the viscosity of the liquid. Specific examples of the liquid include, but are not limited to, liquid medicines such as eye drop and nasal drop.

### Industrial Applicability

The squeeze bottle can be used in such applications as eye dropper, by providing the neck of the squeeze bottle with the delivery member having a valve operated by the liquid pressure that allows liquid drops to be delivered with a small force that can be freely controlled.

## Claims

1. A squeeze bottle comprising a bottle body for containing a liquid, and a neck that leads to an opening end of the bottle body,
wherein the bottle body has a bellows that contracts and expands in a direction generally perpendicular to the direction of delivery of the liquid that is discharged through the neck,
the bellows has a plurality of pleated portions disposed in the direction of delivery,
each of the pleated portions is disposed at intervals from each other in the direction of contraction,
a width of the pleated portion at a convex edge in a direction perpendicular to the direction of contraction is made to decrease from a middle to both ends in the direction of contraction, and
a width of the pleated portion at a concave edge in the direction perpendicular to the direction of contraction is made to decrease from the middle to the both ends in the direction of contraction.

2. The squeeze bottle according to claim 1,
wherein the both edges at the convex edges of the pleated portion in the direction perpendicular to the direction of contraction are disposed generally along a circle when viewed in the direction of delivery, and
the both edges at the concave edges of the pleated portion in the direction perpendicular to the direction of contraction are disposed along a generally concentric circle of a radius smaller than that of the former circle when viewed in the direction of delivery.

3. The squeeze bottle according to claim 1,
wherein the bottle body has a cylindrical portion that adjoins the bellows in the direction of delivery,
the both edges at the convex edges of the pleated portion in the direction perpendicular to the direction of contraction are disposed along a circumferential surface of the cylindrical portion when viewed in the direction of delivery, and
the both edges at the concave edges of the pleated portion in the direction perpendicular to the direction of contraction are disposed along a generally concentric circle of a radius smaller than the circumference of the cylindrical portion when viewed in the direction of delivery.

4. The squeeze bottle according to claim 1 that is formed by blow molding.

5. The squeeze bottle according to claim 1, which is provided with a delivery member that is connected detachably to the neck and has a valve operated by liquid pressure.

6. An eye dropper that utilizes the squeeze bottle according to claim 1.
